# EUROPEAN PATENT APPLICATION

(11) **EP 4 851 278 A1**
(43) Date of publication of application: **09.12.2026**
(21) Application number: 25748377.6
(22) Date of filing: 17.01.2025
(51) Int. Cl.: C12N 1/20, A23L 33/135, C12P 1/04

(54) **PRODUCT OBTAINED THROUGH FERMENTATION OF PLANT-DERIVED COMPOSITION BY LACTIC ACID BACTERIUM BELONGING TO GENUS APILACTOBACILLUS**

(30) Priority: 01.02.2024 JP 2024014267
(71) Applicant: Asahi Group Holdings, Ltd., Tokyo 130-8602 (JP)
(72) Inventor: MORITA Hiroto, Moriya-shi, Ibaraki 302-0106 (JP); YAMASHIRO Eri, Moriya-shi, Ibaraki 302-0106 (JP); ENDO Akihito, Tokyo 156-8502 (JP)
(74) Representative: Hoffmann Eitle
(86) International application number: PCT/JP2025/001419
(87) International publication number: WO 2025/164381

(57) **Abstract**

The present invention provides a lactic acid bacteria fermented product of a plant-derived composition in which the contents of all of glucose, fructose, and sucrose are reduced. The lactic acid bacteria fermented product is obtained by fermenting a plant-derived composition with lactic acid bacteria of the genus Apilactobacillus.

## Description

### Technical Field

The present invention relates to a lactic acid bacteria fermented product which is obtained by fermenting a plant-derived composition with lactic acid bacteria of the genus Apilactobacillus and is characterized by containing mannitol.

### Background Art

Lactic acid bacteria, which have a rich history of consumption and various health functionalities, are used for the fermentation of plant-derived compositions (for example, fruit juices and vegetable juices). For example, fermented fruit juices and the like using Lactobacillus plantarum are known (Patent Literature 1).

In response to the needs for reducing sugars and calories in plant-derived compositions due to growing consumer health consciousness, technologies that utilize the sugar-metabolizing ability (fermentation ability) of lactic acid bacteria to reduce sugars in plant-derived compositions are attracting attention. For example, a technology for reducing the sugar concentration in vegetable or fruit juices using lactic acid bacteria of the genus Leuconostoc is known (Patent Literature 2).

Lactic acid bacteria of the genus Apilactobacillus are a type of fructophilic lactic acid bacteria (FLAB) that consume fructose during glucose metabolism (Non Patent Literature 1).

### Citation List

### Patent Literatures

Patent Literature 1: International Publication No. WO 2011/115114
Patent Literature 2: Japanese Unexamined Patent Application Publication No. 2018-33334

### Non Patent Literatures

Non Patent Literature 1: FEMS Microbiology Letters, 2021, Vol. 368, No. 21-24, fnab150

### Summary of Invention

### Problems to be solved by the invention

The main sugars contained in plant-derived compositions such as fruit juices are glucose, fructose, and sucrose (Standard Tables of Food Composition in Japan 2020 (Eighth Revised Edition)); however, a technology for obtaining a lactic acid bacteria fermented product in which the contents of all three of these types of sugars are simultaneously reduced was not known.

Therefore, an object was set to provide a lactic acid bacteria fermented product of a plant-derived composition in which the contents of all of glucose, fructose, and sucrose (hereinafter, also referred to as "the three sugars") are reduced.

### Means for solution of the problems

As a result of diligent studies to solve this problem, the present inventors found that when lactic acid bacteria of the genus Apilactobacillus metabolize glucose in a plant-derived composition, they consume sucrose in addition to fructose, producing a fermented product in which the aforementioned three types of sugars are simultaneously and significantly reduced. The present invention was made based on this finding.

Specifically, the present invention relates to the following [1] to [15].
[1] A fermented product of a plant-derived composition with lactic acid bacteria of the genus Apilactobacillus, containing mannitol.
[2] The fermented product according to [1], wherein the content of mannitol is 1.5 mg/mL or more.
[3] The fermented product according to [1] or [2], wherein
   a total content (A) of glucose, fructose, and sucrose in the plant-derived composition before fermentation and
   a total content (B) of glucose, fructose, and sucrose in the fermented product have the following relationship:$\left(A-B\right) / A = 0.15 to 1.00$
[4] The fermented product according to any one of [1] to [3], wherein a total content of glucose, fructose, and sucrose is 500 mg/mL or less.
[5] The fermented product according to any one of [1] to [4], wherein the lactic acid bacteria of the genus Apilactobacillus are selected from the group consisting of:
   Apilactobacillus kunkeei,
   Apilactobacillus apinorum,
   Apilactobacillus micheneri, and
   Apilactobacillus timberlakei.
[6] The fermented product according to any one of [1] to [4], wherein the lactic acid bacteria of the genus Apilactobacillus are selected from the group consisting of:
   Apilactobacillus quenuiae,
   Apilactobacillus ozensis,
   Apilactobacillus nanyangensis,
   Apilactobacillus zhangqiuensis, and
   Apilactobacillus xinyiensis.
[7] The fermented product according to any one of [1] to [4], wherein the lactic acid bacteria of the genus Apilactobacillus are Apilactobacillus kunkeei.
[8] The fermented product according to any one of [1] to [4], wherein the lactic acid bacteria of the genus Apilactobacillus are selected from the group consisting of:
   Apilactobacillus kunkeei strain CP3735 (Accession No. NITE BP-04025),
   Apilactobacillus kunkeei strain CP3736 (Accession No. NITE BP-04026),
   Apilactobacillus kunkeei strain CP3737 (Accession No. NITE BP-04027), and
   Apilactobacillus kunkeei strain CP3738 (Accession No. NITE BP-04028).
[9] The fermented product according to any one of [1] to [8], wherein the pH is 5 or less.
[10] The fermented product according to any one of [1] to [9], wherein the plant-derived composition comprises vegetable juice or fruit juice.
[11] The fermented product according to [10], wherein the plant-derived composition comprises apple or orange fruit juice.
[12] A food or drink composition, comprising the fermented product according to any one of [1] to [11].
[13] A fermented product, which is obtained by fermenting a plant-derived composition with lactic acid bacteria, wherein
   the plant-derived composition before fermentation contains glucose, fructose, and sucrose,
   the lactic acid bacteria are lactic acid bacteria of the genus Apilactobacillus, and
   the fermented product contains mannitol.
[14] A method for producing a lactic acid bacteria fermented product of a plant-derived composition, comprising
   a step of fermenting a plant-derived composition containing glucose, fructose, and sucrose with lactic acid bacteria of the genus Apilactobacillus,
   wherein the fermented product contains mannitol.
[15] A method for reducing a sugar content of a plant-derived composition, comprising
   a step of fermenting a plant-derived composition containing a sugar with lactic acid bacteria of the genus Apilactobacillus,
   wherein the sugar is glucose, fructose, and sucrose.

### Advantageous Effects of Invention

The present invention provides a lactic acid bacteria fermented product of a plant-derived composition in which the contents of all three sugars are reduced.

### Description of Embodiments

### [Lactic Acid Bacteria of the Genus Apilactobacillus]

The lactic acid bacteria of the genus Apilactobacillus used in the present invention are not particularly limited as long as they can consume glucose, fructose, and sucrose in a plant-derived composition, and are, for example, the lactic acid bacteria of the following (1) to (9).

### (1) Apilactobacillus kunkeei

Apilactobacillus kunkeei may be any available strain, and may be a type strain (for example, strain JCM 16173^{T}).

Preferred Apilactobacillus kunkeei are strain CP3734, strain CP3735, strain CP3736, strain CP3737, and strain CP3738. As shown in Table 1 below, these strains have been subjected to procedures for international deposition at the National Institute of Technology and Evaluation, NITE Patent Microorganisms Depositary (NPMD) (Room 122, 2-5-8 Kazusakamatari, Kisarazu-shi, Chiba 292-0818, Japan), which is an international depositary authority under the provisions of the Budapest Treaty.

**Table 1**

| Identification indication in the receipt for the original deposit | Receipt number | Date of receipt of the original deposit application |
|---|---|---|
| CP3734 | NITE ABP-04024 | December 1, 2023 |
| CP3735 | NITE ABP-04025 | December 1, 2023 |
| CP3736 | NITE ABP-04026 | December 1, 2023 |
| CP3737 | NITE ABP-04027 | December 1, 2023 |
| CP3738 | NITE ABP-04028 | December 1, 2023 |

The accession numbers issued by the international depositary authority for the strains shown in Table 1 are as follows.

| Identification indication in the receipt for the original deposit | Accession number |
|---|---|
| CP3734 | NITE BP-04024 |
| CP3735 | NITE BP-04025 |
| CP3736 | NITE BP-04026 |
| CP3737 | NITE BP-04027 |
| CP3738 | NITE BP-04028 |

### (2) Apilactobacillus apinorum

Apilactobacillus apinorum may be any available strain, and may be a type strain (for example, strain JCM 30765^{T}) or another strain (for example, strain DSM 26315).

### (3) Apilactobacillus micheneri

Apilactobacillus micheneri may be any available strain, and may be a type strain (for example, strain JCM 33323^{T}) or another strain (for example, strain NBRC 113063).

### (4) Apilactobacillus timberlakei

Apilactobacillus timberlakei may be any available strain, and may be a type strain (for example, strain JCM 33325^{T}).

### (5) Apilactobacillus quenuiae

Apilactobacillus quenuiae may be any available strain, and may be a type strain (for example, strain JCM 33324^{T}).

### (6) Apilactobacillus ozensis

Apilactobacillus ozensis may be any available strain, and may be a type strain (for example, strain JCM 17196^{T}) or another strain (for example, strain JCM 17197, strain JCM 17198, strain JCM 17199, and strain JCM 17200).

### (7) Apilactobacillus nanyangensis

Apilactobacillus nanyangensis may be any available strain, and may be a type strain (for example, strain JCM 33867^{T}).

### (8) Apilactobacillus zhangqiuensis

Apilactobacillus zhangqiuensis may be any available strain, and may be a type strain (for example, strain JCM 34500^{T}).

### (9) Apilactobacillus xinyiensis

Apilactobacillus xinyiensis may be any available strain, and may be a type strain (for example, strain JCM 34501^{T}).

The classification of lactic acid bacteria in this specification follows the reclassification published in 2020. Details of the reclassification are described in Int J Syst Evol Microbiol. 2020 Apr; 70(4): 2782-2858 and on the website of the Japan Bifidus Foundation (URL: https://bifidus-fund.jp/keyword/kw054.shtml), etc.

Known strains belonging to any of the above (1) to (9) and capable of consuming glucose, fructose, and sucrose in a plant-derived composition can be used in the present invention without particular limitation.

Preferred lactic acid bacteria of the genus Apilactobacillus are at least one species selected from the group consisting of the following options, from the viewpoint of the reduction ratio of the three sugars. By using these bacterial species, a reduction ratio of the three sugars of 0.17 or more can be achieved.

Apilactobacillus kunkeei, Apilactobacillus zhangqiuensis, Apilactobacillus ozensis, Apilactobacillus nanyangensis, Apilactobacillus xinyiensis, Apilactobacillus quenuiae, Apilactobacillus apinorum, Apilactobacillus micheneri, and Apilactobacillus timberlakei

More preferred lactic acid bacteria of the genus Apilactobacillus are at least one species selected from the group consisting of the following options. By using these bacterial species, a reduction ratio of the three sugars of 0.20 or more can be achieved.

Apilactobacillus kunkeei, Apilactobacillus zhangqiuensis, Apilactobacillus ozensis, Apilactobacillus nanyangensis, Apilactobacillus xinyiensis, Apilactobacillus quenuiae, and Apilactobacillus apinorum

Particularly preferred lactic acid bacteria of the genus Apilactobacillus are at least one species selected from the group consisting of the following options. By using these bacterial species, a reduction ratio of the three sugars of 0.25 or more can be achieved.

Apilactobacillus kunkeei, Apilactobacillus zhangqiuensis, Apilactobacillus ozensis, Apilactobacillus nanyangensis, Apilactobacillus xinyiensis, and Apilactobacillus quenuiae

Preferred strains of lactic acid bacteria of the genus Apilactobacillus are at least one strain selected from the group consisting of the following options. By using these strains, a reduction ratio of the three sugars of 0.17 or more can be achieved.

Apilactobacillus kunkeei strain CP3735, strain CP3736, strain CP3737, and strain CP3738, Apilactobacillus zhangqiuensis strain JCM 34500^{T}, Apilactobacillus ozensis strain JCM 17198 and strain JCM 17200, Apilactobacillus nanyangensis strain JCM 33867^{T}, Apilactobacillus xinyiensis strain JCM 34501^{T}, Apilactobacillus quenuiae strain JCM 33324^{T}, Apilactobacillus apinorum strain JCM 30765^{T}, Apilactobacillus micheneri strain JCM 33323^{T}, and Apilactobacillus timberlakei strain JCM 33325^{T}

More preferred strains of lactic acid bacteria of the genus Apilactobacillus are at least one strain selected from the group consisting of the following options. By using these strains, a reduction ratio of the three sugars of 0.20 or more can be achieved.

Apilactobacillus kunkeei strain CP3735, strain CP3736, strain CP3737, and strain CP3738, Apilactobacillus zhangqiuensis strain JCM 34500^{T}, Apilactobacillus ozensis strain JCM 17198 and strain JCM 17200, Apilactobacillus nanyangensis strain JCM 33867^{T}, Apilactobacillus xinyiensis strain JCM 34501^{T}, Apilactobacillus quenuiae strain JCM 33324^{T}, and Apilactobacillus apinorum strain JCM 30765^{T}

Particularly preferred strains of lactic acid bacteria of the genus Apilactobacillus are at least one strain selected from the group consisting of the following options. By using these strains, a reduction ratio of the three sugars of 0.25 or more can be achieved.

Apilactobacillus kunkeei strain CP3735, strain CP3736, strain CP3737, and strain CP3738, Apilactobacillus zhangqiuensis strain JCM 34500^{T}, Apilactobacillus ozensis strain JCM 17198 and strain JCM 17200, Apilactobacillus nanyangensis strain JCM 33867^{T}, Apilactobacillus xinyiensis strain JCM 34501^{T}, and Apilactobacillus quenuiae strain JCM 33324^{T}

The lactic acid bacteria of the genus Apilactobacillus may be used as a single type, or a plurality of types may be used in combination.

### [Plant-Derived Composition]

In the present invention, a plant-derived composition is used as a fermentation raw material.

A plant-derived composition refers to a composition containing components derived from plants. The plant-derived composition may be a composition obtained by processing (pressing, etc.) a plant (for example, fruit juice or vegetable juice), or may be a composition containing components isolated from a plant (for example, glucose, fructose, and sucrose) (for example, a lactic acid bacteria medium). The plant-derived composition may further contain components derived from organisms other than plants (for example, yeast extract, beef extract, casein digest, soy digest, in cases where the plant-derived composition is a medium).

The type of plant is not particularly limited and any plant containing glucose, fructose, and sucrose can be used. Specific examples include vegetables, fruits, and the like.

A single type of plant may be used, or a plurality of types may be used in combination (for example, a combination of vegetables and fruits, or a combination of a plurality of types of fruits).

Examples of vegetables include carrots, tomatoes, pumpkins, corn and the like, preferably carrots, tomatoes and pumpkins, and more preferably carrots and tomatoes.

A single type of vegetable may be used, or a plurality of types may be used in combination.

Examples of fruits include apples, citrus fruits, grapes, bananas, strawberries, peaches and the like, preferably apples, citrus fruits and grapes, and more preferably apples and citrus fruits (especially oranges).

A single type of fruit may be used, or a plurality of types may be used in combination.

The means for producing the plant-derived composition is not particularly limited as long as a liquid containing the three sugars can be obtained from a plant. Specific examples include pressing and the like.

The plant-derived composition may be a liquid composition containing solid content (parts of plant organs (for example, fruit) or tissues, seeds, etc.).

The plant-derived composition may be one prepared by dissolving a solid material in water (and further diluting as necessary) (for example, a liquid medium prepared by dissolving a solid medium for lactic acid bacteria containing the three sugars in water and diluting as necessary).

The plant-derived composition may be a concentrated one (for example, concentrated fruit juice or concentrated vegetable juice) or a diluted one.

Examples of the plant-derived composition include fruit juices, vegetable juices, media and the like, preferably fruit juices, and more preferably apple juice and orange juice.

The lower limit of the total content of the three sugars (glucose, fructose, and sucrose) in the plant-derived composition before fermentation is not particularly limited, but is, for example, 1 g/100 mL or more, preferably 4 g/100 mL or more. The upper limit of the total content of the three sugars in the plant-derived composition before fermentation is not particularly limited, but is, for example, 95 g/100 mL or less, preferably 90 g/100 mL or less. Therefore, the range of the total content of the three sugars in the plant-derived composition before fermentation is not particularly limited, but is, for example, 1 to 95 g/100 mL, preferably 4 to 90 g/100 mL.

The lower limit of the glucose content in the plant-derived composition before fermentation is not particularly limited, but is, for example, 1 g/100 mL or more, preferably 2 g/100 mL or more. The upper limit of the glucose content in the plant-derived composition before fermentation is not particularly limited, but is, for example, 45 g/100 mL or less, preferably 35 g/100 mL or less. Therefore, the range of the glucose content in the plant-derived composition before fermentation is not particularly limited, but is, for example, 1 to 45 g/100 mL, preferably 2 to 35 g/100 mL.

The lower limit of the fructose content in the plant-derived composition before fermentation is not particularly limited, but is, for example, 1 g/100 mL or more, preferably 2 g/100 mL or more. The upper limit of the fructose content in the plant-derived composition before fermentation is not particularly limited, but is, for example, 50 g/100 mL or less, preferably 40 g/100 mL or less. Therefore, the fructose content in the plant-derived composition before fermentation is not particularly limited, but is, for example, 1 to 50 g/100 mL, preferably 2 to 40 g/100 mL.

The lower limit of the sucrose content in the plant-derived composition before fermentation is not particularly limited, but is, for example, 0 g/100 mL or more, preferably 1 g/100 mL or more. The upper limit of the sucrose content in the plant-derived composition before fermentation is not particularly limited, but is, for example, 35 g/100 mL or less, preferably 25 g/100 mL or less. Therefore, the sucrose content in the plant-derived composition before fermentation is not particularly limited, but is, for example, 0 to 35 g/100 mL, preferably 1 to 25 g/100 mL.

The Brix value of the plant-derived composition before fermentation is not particularly limited, but is, for example, 1 to 60, preferably 5 to 50.

In order to adjust the sugar content or Brix value, the plant-derived composition before fermentation may be diluted with water, fruit juice, or the like.

The pH of the plant-derived composition before fermentation is not particularly limited, but is, for example, 2 to 10, preferably 4 to 8.

### [Method for Fermenting Plant-Derived Composition]

Fermentation can be carried out using general culture conditions for lactic acid bacteria of the genus Apilactobacillus. The lactic acid bacteria of the genus Apilactobacillus used for fermentation of the fermented product of the present invention can be grown and recovered by culturing using a medium usually used for culturing lactic acid bacteria of the genus Apilactobacillus under commonly used conditions. The lactic acid bacteria of the genus Apilactobacillus used in the present invention may be wet cells or dry cells.

The medium used for culturing the bacterial cells for fermentation usually contains a carbon source, a nitrogen source, inorganic salts, etc., and any medium, either natural or synthetic, may be used as long as it can efficiently culture the lactic acid bacteria of the genus Apilactobacillus. As the carbon source, for example, lactose, glucose, sucrose, fructose, galactose, molasses, and the like can be used. As the nitrogen source, for example, organic nitrogen-containing substances such as casein hydrolysate, whey protein hydrolysate, soy protein hydrolysate, yeast extract, meat extract, and the like can be used. As inorganic salts, for example, phosphate, sodium, potassium, magnesium, manganese, iron, zinc, and the like can be used.

Examples of media suitable for culturing lactic acid bacteria of the genus Apilactobacillus include MRS liquid medium supplemented with 1% fructose, GAM medium, BL medium, Briggs Liver Broth, animal milk, skim milk, milk whey, and the like. Preferably, MRS medium supplemented with 1% fructose can be used.

When used for food applications, a medium composed only of food materials and food additives can also be used. As a medium composed only of food materials (natural medium), tomato juice, carrot juice, other vegetable juices, or apple, orange, pineapple, grape juice, and the like can also be used.

The culture temperature (fermentation temperature) can be, for example, 15 to 45°C, or 20 to 40°C, or about 30°C. The culture temperature can be appropriately adjusted with a constant temperature bath, a mantle heater, a jacket, a fermentation tank, or the like.

Since lactic acid bacteria of the genus Apilactobacillus are facultative anaerobes, culturing (fermentation) may be carried out under aerobic conditions or under anaerobic conditions.

The culture format (fermentation format) is not particularly limited, and examples include static culture, stirred culture, shaking culture, tank culture, and the like.

The culture time (fermentation time) is not particularly limited, but in the case of a culture temperature of 30°C, it can be, for example, 6 to 48 hours, or 8 to 48 hours, or about 20 hours.

In lactic acid bacteria fermentation, lactic acid and the like are produced as fermentation progresses, causing the pH of the fermented product (culture broth) to decrease. Since fermentation by lactic acid bacteria of the genus Apilactobacillus proceeds even under low pH (for example, pH 3 to 4), pH adjustment during fermentation may be omitted.

### [Lactic Acid Bacteria Fermented Product of Plant-Derived Composition]

The fermented product of the present invention is obtained by fermenting the aforementioned plant-derived composition with lactic acid bacteria of the genus Apilactobacillus.

For the fermented product of the present invention, the obtained lactic acid bacteria culture may be used as it is, it may be one containing the cells of the lactic acid bacteria of the genus Apilactobacillus, or it may be one from which the cells have been partially or entirely removed by crude purification by centrifugation or the like and/or solid-liquid separation by filtration or the like.

The degree of fermentation in the fermented product of the present invention is not particularly limited, as long as it satisfies the desired properties (for example, mannitol content, reduction ratio of the three sugars, etc., as described later).

The fermented product of the present invention has the following characteristics.

### [Inclusion of Mannitol]

The fermented product of the present invention contains mannitol (D-mannitol). The mannitol contained in the fermented product is produced exclusively by the lactic acid bacteria of the genus Apilactobacillus.

The lower limit of the mannitol content in the fermented product of the present invention is, for example, 1.5 mg/mL or more, preferably 2 mg/mL or more, more preferably 5 mg/mL or more.

The upper limit of the mannitol content in the fermented product of the present invention is, from the viewpoint of solubility, for example, 213 mg/mL, preferably 100 mg/mL, more preferably 50 mg/mL.

Therefore, the range of the mannitol content in the fermented product of the present invention is, for example, 1.5 to 213 mg/mL, preferably 2 to 213 mg/mL, more preferably 5 to 213 mg/mL, more preferably 5 to 100 mg/mL, and still more preferably 5 to 50 mg/mL.

The content of mannitol can be measured by the HPLC method.

### [Other Characteristics]

The fermented product of the present invention may further have one or more of the following characteristics.

### [Reduction in Content of Glucose, Fructose, and Sucrose (the three sugars)]

Fermentation by lactic acid bacteria of the genus Apilactobacillus proceeds by consuming the three sugars. Therefore, the total content of the three sugars in the fermented product of the present invention is reduced compared to the total content of the three sugars in the plant-derived composition before fermentation.

Specifically, the reduction ratio of the three sugars calculated by the following formula is, for example, 0.15 to 1.00, preferably 0.17 to 1.00, more preferably 0.20 to 1.00, and still more preferably 0.25 to 1.00.

The content of the three sugars can be measured by the HPLC method.$Reduction ratio of the three sugars = \left(A-B\right) / A$
A: Total content of the three sugars in the plant-derived composition before fermentation
B: Total content of the three sugars in the fermented product

The total content of the three sugars in the fermented product of the present invention is, for example, 0 to 500 mg/mL, preferably 0 to 400 mg/mL, more preferably 0 to 350 mg/mL, and still more preferably 0 to 300 mg/mL.

The combination of the "reduction ratio of the three sugars" and the "total content of the three sugars" in the fermented product of the present invention is,
for example, a "reduction ratio of the three sugars" of 0.15 to 1.00 and a "total content of the three sugars" of 0 to 500 mg/mL,
preferably a "reduction ratio of the three sugars" of 0.17 to 1.00 and a "total content of the three sugars" of 0 to 400 mg/mL,
more preferably a "reduction ratio of the three sugars" of 0.20 to 1.00 and a "total content of the three sugars" of 0 to 350 mg/mL,
and still more preferably a "reduction ratio of the three sugars" of 0.25 to 1.00 and a "total content of the three sugars" of 0 to 300 mg/mL.

### [Reduction in Calories]

Along with the aforementioned reduction in the three sugars, the calories of the fermented product of the present invention are reduced compared to the calories of the plant-derived composition before fermentation. Specifically, the calorie reduction ratio calculated by the following formula is preferably 0.05 to 0.6, more preferably 0.1 to 0.5.

The calories in the following formula are the sum of the calories of the three sugars and mannitol. Specifically, the calories can be calculated by quantifying the amounts of the three sugars and mannitol contained in the fermented product or the plant-derived composition before fermentation, and calculating based on the method for calculating energy described in the Standard Tables of Food Composition in Japan 2020 (Eighth Revised Edition), with the three sugars as 3.75 kcal per 1 g and mannitol as 1.6 kcal per 1 g.
Calorie reduction ratio=(A-B)/A
A: Calories of the plant-derived composition before fermentation
B: Calories of the fermented product

### [Reduction in Glucose Content]

The glucose content in the fermented product of the present invention is reduced compared to the glucose content in the plant-derived composition before fermentation. Specifically, the glucose reduction ratio calculated by the following formula is, for example, 0.05 to 1.00, preferably 0.10 to 1.00, more preferably 0.15 to 1.00, still more preferably 0.20 to 1.00, and even more preferably 0.35 to 1.00. The glucose content can be measured by the HPLC method.$Glucose reduction ratio = \left(A-B\right) / A$
A: Glucose content in the plant-derived composition before fermentation
B: Glucose content in the fermented product

### [Reduction in Fructose Content]

The fructose content in the fermented product of the present invention is reduced compared to the fructose content in the plant-derived composition before fermentation. Specifically, the fructose reduction ratio calculated by the following formula is, for example, 0.05 to 1.00, preferably 0.10 to 1.00, more preferably 0.15 to 1.00, still more preferably 0.20 to 1.00, and even more preferably 0.25 to 1.00.

The fructose content can be measured by the HPLC method.$Fructose reduction ratio = \left(A-B\right) / A$
A: Fructose content in the plant-derived composition before fermentation
B: Fructose content in the fermented product

### [Reduction in Sucrose Content]

The sucrose content in the fermented product of the present invention is reduced compared to the sucrose content in the plant-derived composition before fermentation. Specifically, the sucrose reduction ratio calculated by the following formula is, for example, 0.01 to 1.00, preferably 0.02 to 1.00, more preferably 0.03 to 1.00, still more preferably 0.04 to 1.00, and even more preferably 0.05 to 1.00.

The sucrose content can be measured according to the HPLC method.$Sucrose reduction ratio = \left(A-B\right) / A$
A: Sucrose content in the plant-derived composition before fermentation
B: Sucrose content in the fermented product

### [pH]

The pH of the fermented product of the present invention is preferably 5 or less from the viewpoint of flavor.

### [Brix]

The Brix value of the fermented product of the present invention is preferably 1 to 50, more preferably 1 to 30.

### [Inclusion of Solid Content]

When a plant-derived composition containing solid content is used as a fermentation raw material, the fermented product of the present invention may contain solid content. The solid content in the fermented product is, for example, 90% by mass or less, preferably 80% by mass or less, more preferably 70% by mass or less.

### [Food and Drink Composition]

In one embodiment of the present invention, the fermented product is used as it is as a food and drink (food and beverage). The form of the food or drink is not particularly limited as long as it can be orally ingested. Specific examples include liquid beverages and solid foods (for example, jelly). The fermented product can also be dried to form a powder or granules. Specific drying methods are not particularly limited, but examples include spray drying, drum drying, vacuum drying, freeze drying, and the like, and these can be adopted alone or in combination. At that time, a commonly used carrier or excipient may be added as necessary.

In one embodiment of the present invention, the fermented product is combined with optional ingredients and used as a food or drink composition. The form of the food or drink composition is not particularly limited as long as it can be orally ingested. Specific examples include liquid beverages. In this case, the liquid beverage may be a beverage for dilution (to be ingested after being diluted with water or the like). The food or drink composition may be a solid (for example, jelly). The food or drink composition may be dried to form a powder or granules, and further, if necessary, may be formed into granules, tablets, or capsules.

As for optional ingredients, ingredients that can be added to food and drink can be used without particular limitation. For example, additives for liquid beverages include pH adjusters, emulsifiers, stabilizers, fragrances, and the like.

The optional ingredient may be a functional ingredient (for example, sterilized lactic acid bacteria cells, peptides, etc.). Depending on the type of functional ingredient added, the food or drink composition may be a functional food or drink.

Functional food and drink include all so-called health food and drink. Examples of functional food and drink include Foods with Health Claims (Foods for Specified Health Uses (including conditional FOSHU), Foods with Function Claims, and Foods with Nutrient Function Claims), Foods for Special Dietary Uses, dietary supplements, health supplements, supplements, and beauty food and drink. Functional food and drink include health food and drink to which a health claim based on the food standards of the Codex Alimentarius Commission (FAO/WHO Joint Food Standards Programme) is applied.

### [Method for Reducing Sugar Content of a Plant-Derived Composition Juice]

The method for obtaining a fermented product of a plant-derived composition with a reduced content of the three sugars as described above can also be understood as a "method for reducing the sugar content of a plant-derived composition." This reduction method includes a step of fermenting a plant-derived composition containing the three sugars with lactic acid bacteria of the genus Apilactobacillus. This fermentation step can be carried out under the culture conditions described in the aforementioned [Method for Fermenting Plant-Derived Composition].

Another aspect of the present invention may be as follows.
<1> A fermented product of a plant-derived composition with lactic acid bacteria of the genus Apilactobacillus, containing mannitol, wherein
   the content of mannitol is 1.5 mg/mL or more, and
   the lactic acid bacteria of the genus Apilactobacillus are selected from the group consisting of:
      Apilactobacillus kunkeei,
      Apilactobacillus apinorum,
      Apilactobacillus micheneri, and
      Apilactobacillus timberlakei, or
   the lactic acid bacteria of the genus Apilactobacillus are selected from the group consisting of:
      Apilactobacillus quenuiae,
      Apilactobacillus ozensis,
      Apilactobacillus nanyangensis,
      Apilactobacillus zhangqiuensis, and
      Apilactobacillus xinyiensis, or
   the lactic acid bacteria of the genus Apilactobacillus are Apilactobacillus kunkeei.
<2> A fermented product of a plant-derived composition with lactic acid bacteria of the genus Apilactobacillus, containing mannitol, wherein
   the content of mannitol is 1.5 mg/mL or more, and
   the plant-derived composition comprises apple or orange fruit juice.
<3> A fermented product of a plant-derived composition with lactic acid bacteria of the genus Apilactobacillus, containing mannitol, wherein
   the lactic acid bacteria of the genus Apilactobacillus are selected from the group consisting of:
      Apilactobacillus kunkeei,
      Apilactobacillus apinorum,
      Apilactobacillus micheneri, and
      Apilactobacillus timberlakei, or
   the lactic acid bacteria of the genus Apilactobacillus are selected from the group consisting of:
      Apilactobacillus quenuiae,
      Apilactobacillus ozensis,
      Apilactobacillus nanyangensis,
      Apilactobacillus zhangqiuensis, and
      Apilactobacillus xinyiensis, or
   the lactic acid bacteria of the genus Apilactobacillus are Apilactobacillus kunkeei, and
   the plant-derived composition comprises apple or orange fruit juice.
<4> A fermented product of a plant-derived composition with lactic acid bacteria of the genus Apilactobacillus, containing mannitol, wherein
   the content of mannitol is 1.5 mg/mL or more, and
   the lactic acid bacteria of the genus Apilactobacillus are selected from the group consisting of:
      Apilactobacillus kunkeei,
      Apilactobacillus apinorum,
      Apilactobacillus micheneri, and
      Apilactobacillus timberlakei, or
   the lactic acid bacteria of the genus Apilactobacillus are selected from the group consisting of:
      Apilactobacillus quenuiae,
      Apilactobacillus ozensis,
      Apilactobacillus nanyangensis,
      Apilactobacillus zhangqiuensis, and
      Apilactobacillus xinyiensis, or
   the lactic acid bacteria of the genus Apilactobacillus are Apilactobacillus kunkeei, and
   the plant-derived composition comprises apple or orange fruit juice.
<5> A fermented product of a plant-derived composition with lactic acid bacteria of the genus Apilactobacillus, containing mannitol, wherein
   the content of mannitol is 1.5 mg/mL or more, and
   a total content (A) of glucose, fructose, and sucrose in the plant-derived composition before fermentation and
   a total content (B) of glucose, fructose, and sucrose in the fermented product
   have the following relationship:$\left(A-B\right) / A = 0.15 to 1.00 .$
<6> A fermented product of a plant-derived composition with lactic acid bacteria of the genus Apilactobacillus, containing mannitol, wherein
   the content of mannitol is 1.5 mg/mL or more, and
   a total content of glucose, fructose, and sucrose is 500 mg/mL or less.
<7> A fermented product of a plant-derived composition with lactic acid bacteria of the genus Apilactobacillus, containing mannitol, wherein
   the lactic acid bacteria of the genus Apilactobacillus are selected from the group consisting of:
      Apilactobacillus kunkeei,
      Apilactobacillus apinorum,
      Apilactobacillus micheneri, and
      Apilactobacillus timberlakei, or
   the lactic acid bacteria of the genus Apilactobacillus are selected from the group consisting of:
      Apilactobacillus quenuiae,
      Apilactobacillus ozensis,
      Apilactobacillus nanyangensis,
      Apilactobacillus zhangqiuensis, and
      Apilactobacillus xinyiensis, or
   the lactic acid bacteria of the genus Apilactobacillus are Apilactobacillus kunkeei, and
   a total content (A) of glucose, fructose, and sucrose in the plant-derived composition before fermentation and
   a total content (B) of glucose, fructose, and sucrose in the fermented product
   have the following relationship: $\left(A-B\right) / A = 0.15 to 1.00 .$
<8> A fermented product of a plant-derived composition with lactic acid bacteria of the genus Apilactobacillus, containing mannitol, wherein
   the lactic acid bacteria of the genus Apilactobacillus are selected from the group consisting of:
      Apilactobacillus kunkeei,
      Apilactobacillus apinorum,
      Apilactobacillus micheneri, and
      Apilactobacillus timberlakei, or
   the lactic acid bacteria of the genus Apilactobacillus are selected from the group consisting of:
      Apilactobacillus quenuiae,
      Apilactobacillus ozensis,
      Apilactobacillus nanyangensis,
      Apilactobacillus zhangqiuensis, and
      Apilactobacillus xinyiensis, or
   the lactic acid bacteria of the genus Apilactobacillus are Apilactobacillus kunkeei, and
   a total content of glucose, fructose, and sucrose is 500 mg/mL or less.
<9> A fermented product, which is obtained by fermenting a plant-derived composition with lactic acid bacteria, wherein
   the plant-derived composition before fermentation contains glucose, fructose, and sucrose,
   the lactic acid bacteria are lactic acid bacteria of the genus Apilactobacillus,
   the fermented product contains mannitol, and
   the content of mannitol is 1.5 mg/mL or more.
<10> The fermented product according to <9>, wherein
   a total content (A) of glucose, fructose, and sucrose in the plant-derived composition before fermentation and
   a total content (B) of glucose, fructose, and sucrose in the fermented product
   have the following relationship: $\left(A-B\right) / A = 0.15 to 1.00 .$
<11> The fermented product according to <9> or <10>, wherein a total content of glucose, fructose, and sucrose is 500 mg/mL or less.
<12> The fermented product according to any one of <9> to <11>, wherein the lactic acid bacteria of the genus Apilactobacillus are selected from the group consisting of:
   Apilactobacillus kunkeei,
   Apilactobacillus apinorum,
   Apilactobacillus micheneri, and
   Apilactobacillus timberlakei.
<13> The fermented product according to any one of <9> to <11>, wherein the lactic acid bacteria of the genus Apilactobacillus are selected from the group consisting of:
   Apilactobacillus quenuiae,
   Apilactobacillus ozensis,
   Apilactobacillus nanyangensis,
   Apilactobacillus zhangqiuensis, and
   Apilactobacillus xinyiensis.
<14> The fermented product according to any one of <9> to <11>, wherein the lactic acid bacteria of the genus Apilactobacillus are Apilactobacillus kunkeei.
<15> The fermented product according to any one of <9> to <11>, wherein the lactic acid bacteria of the genus Apilactobacillus are selected from the group consisting of:
   Apilactobacillus kunkeei strain CP3735 (Accession No. NITE BP-04025),
   Apilactobacillus kunkeei strain CP3736 (Accession No. NITE BP-04026),
   Apilactobacillus kunkeei strain CP3737 (Accession No. NITE BP-04027), and
   Apilactobacillus kunkeei strain CP3738 (Accession No. NITE BP-04028).
<16> The fermented product according to any one of <9> to <15>, wherein the pH is 5 or less.
<17> The fermented product according to any one of <9> to <16>, wherein the plant-derived composition comprises vegetable juice or fruit juice.
<18> The fermented product according to <17>, wherein the plant-derived composition comprises apple or orange fruit juice.
<19> A method for producing a lactic acid bacteria fermented product of a plant-derived composition, comprising
   a step of fermenting a plant-derived composition containing glucose, fructose, and sucrose with lactic acid bacteria of the genus Apilactobacillus,
   wherein the fermented product contains mannitol, and
   the content of mannitol is 1.5 mg/mL or more.
<20> The method according to <19>, wherein
   a total content (A) of glucose, fructose, and sucrose in the plant-derived composition before fermentation and
   a total content (B) of glucose, fructose, and sucrose in the fermented product
   have the following relationship: $\left(A-B\right) / A = 0.15 to 1.00 .$
<21> The method according to <19> or <20>, wherein a total content of glucose, fructose, and sucrose in the lactic acid bacteria fermented product is 500 mg/mL or less.
<22> The method according to any one of <19> to <21>, wherein the lactic acid bacteria of the genus Apilactobacillus are selected from the group consisting of:
   Apilactobacillus kunkeei,
   Apilactobacillus apinorum,
   Apilactobacillus micheneri, and
   Apilactobacillus timberlakei.
<23> The method according to any one of <19> to <21>, wherein the lactic acid bacteria of the genus Apilactobacillus are selected from the group consisting of:
   Apilactobacillus quenuiae,
   Apilactobacillus ozensis,
   Apilactobacillus nanyangensis,
   Apilactobacillus zhangqiuensis, and
   Apilactobacillus xinyiensis.
<24> The method according to any one of <19> to <21>, wherein the lactic acid bacteria of the genus Apilactobacillus are Apilactobacillus kunkeei.
<25> The method according to any one of <9> to <21>, wherein the lactic acid bacteria of the genus Apilactobacillus are selected from the group consisting of:
   Apilactobacillus kunkeei strain CP3735 (Accession No. NITE BP-04025),
   Apilactobacillus kunkeei strain CP3736 (Accession No. NITE BP-04026),
   Apilactobacillus kunkeei strain CP3737 (Accession No. NITE BP-04027), and
   Apilactobacillus kunkeei strain CP3738 (Accession No. NITE BP-04028).
<26> The method according to any one of <19> to <25>, wherein the pH of the lactic acid bacteria fermented product is 5 or less.
<27> The method according to any one of <9> to <26>, wherein the plant-derived composition comprises vegetable juice or fruit juice.
<28> The method according to <27>, wherein the plant-derived composition comprises apple or orange fruit juice.
<29> A method for reducing a sugar content of a plant-derived composition, comprising
   a step of fermenting a plant-derived composition containing a sugar with lactic acid bacteria of the genus Apilactobacillus,
   wherein the sugar is glucose, fructose, and sucrose, and
   a mannitol content of the lactic acid bacteria fermented product of the plant-derived composition is 1.5 mg/mL or more.
<30> The method according to <29>, wherein
   a total content (A) of glucose, fructose, and sucrose in the plant-derived composition before fermentation and
   a total content (B) of glucose, fructose, and sucrose in the lactic acid bacteria fermented product of the plant-derived composition
   have the following relationship: $\left(A-B\right) / A = 0.15 to 1.00 .$
<31> The method according to <29> or <30>, wherein a total content of glucose, fructose, and sucrose in the lactic acid bacteria fermented product of the plant-derived composition is 500 mg/mL or less.
<32> The method according to any one of <29> to <31>, wherein the lactic acid bacteria of the genus Apilactobacillus are selected from the group consisting of:
   Apilactobacillus kunkeei,
   Apilactobacillus apinorum,
   Apilactobacillus micheneri, and
   Apilactobacillus timberlakei.
<33> The method according to any one of <29> to <31>, wherein the lactic acid bacteria of the genus Apilactobacillus are selected from the group consisting of:
   Apilactobacillus quenuiae,
   Apilactobacillus ozensis,
   Apilactobacillus nanyangensis,
   Apilactobacillus zhangqiuensis, and
   Apilactobacillus xinyiensis.
<34> The method according to any one of <29> to <31>, wherein the lactic acid bacteria of the genus Apilactobacillus are Apilactobacillus kunkeei.
<35> The method according to any one of <29> to <31>, wherein the lactic acid bacteria of the genus Apilactobacillus are selected from the group consisting of:
   Apilactobacillus kunkeei strain CP3735 (Accession No. NITE BP-04025),
   Apilactobacillus kunkeei strain CP3736 (Accession No. NITE BP-04026),
   Apilactobacillus kunkeei strain CP3737 (Accession No. NITE BP-04027), and
   Apilactobacillus kunkeei strain CP3738 (Accession No. NITE BP-04028).
<36> The method according to any one of <29> to <35>, wherein the pH of the lactic acid bacteria fermented product of the plant-derived composition is 5 or less.
<37> The method according to any one of <29> to <36>, wherein the plant-derived composition comprises vegetable juice or fruit juice.
<38> The method according to <37>, wherein the plant-derived composition comprises apple or orange fruit juice.

### Examples

Next, the effects of the present invention will be specifically described by way of examples, but the present invention is not limited to these examples.

The culturing of lactic acid bacteria in all examples was conducted under aerobic conditions.

### [Example 1: Fermentation of Fruit Juice Using Various Lactic Acid Bacteria]

### [Test Strains]

The lactic acid bacteria strains shown in Table 2 were used.

**Table 2**

| Bacterial Species | Strain |
|---|---|
| Apilactobacillus kunkeei | CP3735 (Accession No.: NITE BP-04025) |
| Apilactobacillus kunkeei | CP3736 (Accession No.: NITE BP-04026) |
| Apilactobacillus kunkeei | JCM 16173^{T} (Type strain) |
| Fructobacillus fructosus | NRIC 1058^{T} (Type strain) |
| Lactiplantibacillus plantarum | NRIC 1067^{T} (Type strain) |
| Leuconostoc gelidum | NRIC 1778^{T} (Type strain) |
| Leuconostoc citreum | NRIC 1776^{T} (Type strain) |
| Oenococcus oeni | NRIC 645^{T} (Type strain) |

Among the lactic acid bacteria other than the genus Apilactobacillus (used as comparative examples), Fructobacillus fructosus was a fructophilic lactic acid bacterium, similar to the lactic acid bacteria of the genus Apilactobacillus, but the other lactic acid bacteria were non-fructophilic lactic acid bacteria.

### [Plant-Derived Composition (Fermentation Raw Material)]

Fermentation raw materials were prepared from concentrated apple juice and concentrated orange juice.

Concentrated apple juice (from Italy) was diluted with distilled water so that the Brix value became 10. To the diluted apple juice, Yeast Extract (Becton, Dickinson and Company) was added as a fermentation aid (final concentration 0.5%), the pH was adjusted to 6.0 with 1N NaOH, and the mixture was subjected to sterile filtration (filter pore size: 0.22 µm) to be used as a fermentation raw material. The pH was measured using a pH meter (Compact PH Meter LAQU, HORIBA, Ltd.).

Concentrated orange juice (from Brazil) was diluted with distilled water so that the Brix value became 10. Without adding Yeast Extract, the pH of the diluted orange juice was adjusted to 6.0 with 1N NaOH, and the mixture was subjected to sterile filtration (filter pore size: 0.22 µm) to be used as a fermentation raw material.

The contents of the three sugars (glucose, fructose, and sucrose) and mannitol, pH, and Brix value in each fermentation raw material are shown in Table 3. "The three sugars" in the table indicates the total content of glucose, fructose, and sucrose.

**Table 3**

| Plant-Derived Composition (Fermentation Raw Material) | Content (g/100mL) | | | | Mannitol (mg/mL) | Brix Value | pH |
|---|---|---|---|---|---|---|---|
| | Sucrose | Glucose | Fructose | The three sugars | | | |
| Apple juice | 1.29 | 2.15 | 5.66 | 9.10 | 0.00 | 10 | 6.0 |
| Orange juice | 3.61 | 2.02 | 2.20 | 7.84 | 0.00 | 10 | 6.0 |

### [Culturing of Bacterial Cells for Fermentation]

Each lactic acid bacteria strain was inoculated into Lactobacillus MRS Broth (Becton, Dickinson and Company) supplemented with 1% fructose in a 1.5 mL tube and statically cultured at 30°C for 20 hours. The bacterial cells collected by centrifugation (3500 x g, 10 minutes) were washed twice with physiological saline (0.8% NaCl). The washed cells were collected by centrifugation (3500 x g, 10 minutes) and suspended in a volume of physiological saline equal to that of the medium used for culturing. The resulting lactic acid bacteria suspension was used for the fermentation of each fermentation raw material.

### [Lactic Acid Bacteria Fermentation of Apple Juice]

To 1 mL of the aforementioned sterile apple juice, 50 µL of the lactic acid bacteria suspension was added (inoculation amount is 5% relative to the volume of the sterile apple juice), and static fermentation was performed at 37°C for 18 to 22 hours to obtain fermented apple juice.

### [Lactic Acid Bacteria Fermentation of Orange Juice]

To 1 mL of the aforementioned sterile orange juice, 50 µL of the lactic acid bacteria suspension was added (inoculation amount is 5% relative to the volume of the sterile orange juice), and static fermentation was performed at 37°C for 18 to 22 hours to obtain fermented orange juice.

The pH of each fermented fruit juice was measured. Furthermore, the sugar content of each fermented fruit juice was measured according to the following procedure.

### [Measurement of Sugar Content of Fermented Fruit Juice]

The supernatant obtained by subjecting the fermented fruit juice to centrifugation was diluted with distilled water so that the Brix value became 1 (10-fold dilution when the fruit juice used as the fermentation raw material had a Brix value of 10). The diluted solution was filtered to remove impurities (filter pore size: 0.45 µm). The respective contents of sucrose, glucose, fructose and mannitol in the filtrate were measured by high-performance liquid chromatography (HPLC). The HPLC measurement was performed using the model: Nexcera-i LC-2040C (Shimadzu Corporation), detector: Refractive Index Detector RID-20A (Shimadzu Corporation), and column: SUGAR SP0810 (Shodex). The analysis was conducted with the mobile phase: ultrapure water, flow rate: 0.5 mL/min, column temperature: 80°C, and sample injection volume: 10 µL.

In addition, the contents of sucrose, glucose, fructose and mannitol of the unfermented fruit juice (prepared according to the same method as the fermented fruit juice but except for the addition of the lactic acid bacteria suspension; hereinafter, also referred to as 'pre-fermentation fruit juice') were quantified by the same method.

The measured values were applied to the following calculation formula to calculate the reduction ratio for each of sucrose, glucose, fructose, and the three sugars (sucrose, glucose, fructose).$Reduction ratio = \left(A-B\right) / A$
A: Sugar content of the pre-fermentation fruit juice
B: Sugar content of the fermented fruit juice

The results for the fermented apple juice are shown in Table 4.

The results for the fermented orange juice are shown in Table 5.

Since fermentation was performed three times for each strain, the "Reduction ratio," "Mannitol content" and "pH" in Tables 4 to 5 are the average values of three measurements or calculations (n=3).

**Table 4**

| Fermented Apple Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei CP3735 | 0.48 | 0.64 | 0.44 | 0.49 | 46.36 | 30.01 | 3.80 |
| Apilactobacillus kunkeei CP3736 | 0.67 | 0.60 | 0.38 | 0.48 | 47.74 | 28.24 | 3.83 |
| Apilactobacillus kunkeei JCM 16173^{T} | 0.73 | 0.52 | 0.28 | 0.40 | 54.59 | 23.40 | 3.90 |
| Fructobacillus fructosus | -0.07 | 0.45 | 0.30 | 0.28 | 65.17 | 18.81 | 4.07 |
| Lactiplantibacillus plantarum | -0.06 | 0.26 | 0.05 | 0.08 | 83.60 | 0.00 | 3.80 |
| Leuconostoc gelidum | -0.05 | 0.13 | 0.01 | 0.03 | 88.36 | 1.37 | 4.90 |
| Leuconostoc citreum | -0.07 | 0.25 | 0.04 | 0.07 | 84.26 | 0.00 | 3.80 |
| Oenococcus oeni | -0.05 | 0.05 | 0.03 | 0.02 | 89.04 | 1.32 | 5.70 |

**Table 5**

| Fermented Orange Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei CP3735 | 0.40 | 0.58 | 0.80 | 0.56 | 34.56 | 24.46 | 4.07 |
| Apilactobacillus kunkeei CP3736 | 0.52 | 0.61 | 0.72 | 0.60 | 31.27 | 24.66 | 4.10 |
| Apilactobacillus kunkeei JCM 16173^{T} | 0.91 | 0.51 | 0.24 | 0.62 | 29.87 | 21.46 | 4.20 |
| Fructobacillus fructosus | 0.05 | 0.51 | 0.58 | 0.31 | 53.85 | 13.49 | 4.67 |
| Lactiplantibacillus plantarum | 0.01 | 0.34 | 0.12 | 0.13 | 68.52 | 0.31 | 4.17 |
| Leuconostoc gelidum | 0.02 | 0.14 | 0.04 | 0.05 | 74.16 | 0.64 | 5.17 |
| Leuconostoc citreum | -0.01 | 0.34 | 0.13 | 0.12 | 69.09 | 0.35 | 4.10 |
| Oenococcus oeni | 0.96 | -0.74 | -0.38 | 0.10 | 70.27 | 0.80 | 6.00 |

### [Example 2: Fermentation of Fruit Juice Using Various Lactic Acid Bacteria of the Genus Apilactobacillus]

### [Test Strains]

The lactic acid bacteria strains of the genus Apilactobacillus shown in Table 6 were used.

**Table 6**

| Bacterial Species | Strain |
|---|---|
| Apilactobacillus kunkeei | JCM 16173^{T} (Type strain) |
| Apilactobacillus apinorum | JCM 30765^{T} (Type strain) |
| Apilactobacillus micheneri | JCM 33323^{T} (Type strain) |
| Apilactobacillus nanyangensis | JCM 33867^{T} (Type strain) |
| Apilactobacillus ozensis | JCM 17198 |
| Apilactobacillus ozensis | JCM 17200 |
| Apilactobacillus quenuiae | JCM 33324^{T} (Type strain) |
| Apilactobacillus timberlakei | JCM 33325^{T} (Type strain) |
| Apilactobacillus xinyiensis | JCM 34501^{T} (Type strain) |
| Apilactobacillus zhangqiuensis | JCM 34500^{T} (Type strain) |

### [Plant-Derived Composition (Fermentation Raw Material)]

A fermentation raw material (pH 6.0, Brix value 10) was prepared from concentrated apple juice according to the method described in Example 1.

A fermentation raw material (pH 6.0, Brix value 10) was prepared from concentrated orange juice according to the method described in Example 1.

### [Lactic Acid Bacteria Fermentation]

Using each lactic acid bacterium of the genus Apilactobacillus, each fermentation raw material was fermented by the same method as in Example 1. Thereafter, the pH and sugar content of the fermented fruit juice were measured by the same method as in Example 1.

The results for the fermented apple juice are shown in Table 7.

The results for the fermented orange juice are shown in Table 8.

Since fermentation was performed three times for each strain, the "Reduction ratio," "Mannitol content" and "pH" in Tables 7 to 8 are the average values of three measurements or calculations (n=3).

**Table 7**

| Fermented Apple Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei | 0.29 | 0.36 | 0.24 | 0.28 | 75.95 | 14.16 | 4.08 |
| Apilactobacillus apinorum | 0.07 | *0.34* | 0.27 | 0.24 | 80.09 | 14.11 | 4.01 |
| Apilactobacillus micheneri | 0.04 | 0.26 | 0.22 | 0.19 | 85.34 | 10.76 | 4.17 |
| Apilactobacillus nanyangensis | 0.03 | 0.52 | 0.36 | 0.32 | 71.39 | 20.94 | 3.95 |
| Apilactobacillus ozensis JCM 17198 | 0.06 | 0.61 | 0.45 | 0.40 | 63.12 | 25.30 | 3.63 |
| Apilactobacillus ozensis JCM 17200 | 0.02 | 0.60 | 0.44 | 0.38 | 65.02 | 25.69 | 3.63 |
| Apilactobacillus quenuiae | 0.06 | 0.39 | 0.30 | 0.27 | 77.36 | 14.72 | 4.08 |
| Apilactobacillus timberlakei | 0.10 | 0.22 | 0.18 | 0.17 | 87.37 | 7.22 | 4.50 |
| Apilactobacillus xinyiensis | 0.08 | 0.55 | 0.42 | 0.37 | 66.15 | 22.38 | 3.67 |
| Apilactobacillus zhangqiuensis | 0.38 | 0.51 | 0.34 | 0.39 | 64.68 | 21.05 | 3.79 |

**Table 8**

| Fermented Orange Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei | 0.96 | 0.53 | 0.24 | 0.63 | 33.17 | *22.30* | 4.28 |
| Apilactobacillus apinorum | 0.12 | 0.49 | 0.79 | 0.42 | 51.04 | 20.83 | 4.24 |
| Apilactobacillus micheneri | 0.07 | 0.25 | 0.38 | 0.21 | 69.83 | 8.88 | 4.86 |
| Apilactobacillus nanyangensis | 0.06 | 0.68 | 0.91 | 0.49 | 45.60 | 23.51 | 4.17 |
| Apilactobacillus ozensis JCM 17198 | 0.05 | 0.63 | 0.88 | 0.46 | 48.03 | 22.54 | 4.17 |
| Apilactobacillus ozensis JCM 17200 | 0.06 | 0.63 | 0.89 | 0.46 | 47.70 | 22.56 | 4.17 |
| Apilactobacillus quenuiae | 0.07 | 0.38 | 0.55 | 0.30 | 62.31 | 15.10 | 4.62 |
| Apilactobacillus timberlakei | 0.09 | 0.21 | 0.33 | 0.20 | 71.22 | 7.86 | 4.94 |
| Apilactobacillus xinyiensis | 0.07 | 0.63 | 0.92 | 0.48 | 45.97 | 23.50 | 4.13 |
| Apilactobacillus zhangqiuensis | 0.81 | 0.58 | 0.41 | 0.63 | 33.22 | 23.29 | 4.09 |

### [Example 3: Fermentation of Fruit Juice (High Brix Value) Using Various Lactic Acid Bacteria]

### [Test Strains]

The lactic acid bacteria strains shown in Table 9 were used.

**Table 9**

| Bacterial Species | Strain |
|---|---|
| Apilactobacillus kunkeei | JCM 16173^{T} (Type strain) |
| Apilactobacillus kunkeei | CP3735 (Accession No.: NITE BP-04025) |
| Apilactobacillus kunkeei | CP3736 (Accession No.: NITE BP-04026) |
| Lactiplantibacillus plantarum | NRIC1067^{T} (Type strain) |
| Leuconostoc citreum | NRIC1776^{T} (Type strain) |

### [Plant-Derived Composition (Fermentation Raw Material)]

Fermentation raw materials were prepared from concentrated apple juice and concentrated orange juice.

Concentrated apple juice (from Italy) was diluted so that the Brix value became 30. To this apple juice, Yeast Extract (Becton, Dickinson and Company) was added as a fermentation aid (final concentration 0.5%), the pH was adjusted to 6.0 with 1N NaOH, and the mixture was subjected to sterile filtration (filter pore size: 0.22 µm) to be used as a fermentation raw material.

Concentrated orange juice (from Brazil) was diluted so that the Brix value became 40. Without adding yeast extract to this orange juice, the pH was adjusted to 6.0 with 1N NaOH, and the mixture was subjected to sterile filtration (filter pore size: 0.22 µm) to be used as a fermentation raw material.

The contents of the three sugars (glucose, fructose, and sucrose) and mannitol, pH and Brix value in each fermentation raw material are shown in Table 10.

**Table 10**

| Plant-Derived Composition (Fermentation Raw Material) | Content (g/100ml) | | | | Mannitol (mg/mL) | Brix Value | pH |
|---|---|---|---|---|---|---|---|
| | Sucrose | Glucose | Fructose | The three sugars | | | |
| Apple juice | 3.37 | 5.84 | 15.34 | 24.55 | 0.00 | 30 | 6.0 |
| Orange juice | 10.99 | 6.82 | 7.93 | 25.73 | 0.00 | 40 | 6.0 |

Using each lactic acid bacterium, the aforementioned fermentation raw materials were fermented by the same method as in Example 1. Thereafter, the pH and sugar content of the fermented fruit juice were measured by the same method as in Example 1.

The results for the fermented apple juice are shown in Table 11.

The results for the fermented orange juice are shown in Table 12.

Since fermentation was performed three times for each strain, the "Reduction ratio," "Mannitol content" and "pH" in Tables 11 to 12 are the average values of three measurements or calculations (n=3).

**Table 11**

| Fermented Apple Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei JCM 16173^{T} | 0.81 | 0.34 | 0.18 | 0.31 | 171.2 | 36.40 | 4.20 |
| Apilactobacillus kunkeei CP3735 | 0.37 | 0.35 | 0.25 | 030 | 173.6 | 41.11 | 4.10 |
| Apilactobacillus kunkeei CP3736 | 0.54 | 0.34 | 0.23 | 0.31 | 171.9 | 41.25 | 4.10 |
| Lactiplantibacillus plantarum | 0.01 | 0.18 | 0.03 | 0.06 | 230.9 | 0.00 | 4.37 |
| Leuconostoc citreum | 0.15 | 0.08 | 0.06 | 0.08 | 225.9 | 4.40 | 5.00 |

**Table 12**

| Fermented Orange Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei JCM 16173^{T} | 0.22 | 0.12 | 0.06 | 0.15 | 220.1 | 13.05 | 5.30 |
| Apilactobacillus kunkeei CP3735 | 0.16 | 0.25 | 0.43 | 0.27 | 188.8 | 39.87 | 4.60 |
| Apilactobacillus kunkeei CP3736 | 0.12 | 0.29 | 0.41 | 0.25 | 192.6 | 41.45 | 4.63 |
| Lactiplantibacillus plantarum | -0.03 | 0.12 | -0.03 | 0.01 | 255.1 | 0.00 | 5.00 |
| Leuconostoc citreum | 0.02 | 0.06 | 0.13 | 0.06 | 241.6 | 11.47 | 5.20 |

### [Example 4: Fermentation of Fruit Juice Under Low pH Using Various Lactic Acid Bacteria]

### [Test Strains]

The same lactic acid bacteria strains as in Example 3 were used.

### [Plant-Derived Composition (Fermentation Raw Material)]

Fermentation raw materials were prepared from concentrated apple juice and concentrated orange juice.

Concentrated apple juice (from Italy) was diluted with distilled water so that the Brix value became 10. Yeast Extract (Becton, Dickinson and Company) was added as a fermentation aid (final concentration 0.5%), the pH was adjusted to 5.0 with 1N NaOH, and the mixture was subjected to sterile filtration (filter pore size: 0.22 µm) to be used as a fermentation raw material.

Concentrated orange juice (from Brazil) was diluted with distilled water so that the Brix value became 10. Without adding yeast extract to the diluted orange juice, the pH was adjusted to 4.0 with 1N NaOH, and the mixture was subjected to sterile filtration (filter pore size: 0.22 µm) to be used as a fermentation raw material.

The contents of the three sugars (glucose, fructose, and sucrose) and mannitol, pH and Brix value in each fermentation raw material are shown in Table 13.

**Table 13**

| Plant-Derived Composition (Fermentation Raw Material) | Content (g/100mL) | | | | Mannitol (mg/mL) | Brix Value | pH |
|---|---|---|---|---|---|---|---|
| | Sucrose | Glucose | Fructose | The three sugars | | | |
| Apple juice | 1.37 | 2.56 | 6.46 | 10.39 | 0.00 | 10 | 5.0 |
| Orange juice | 3.21 | 2.05 | 2.41 | 7.67 | 0.00 | 10 | 4.0 |

Using each lactic acid bacterium, the aforementioned fermentation raw materials were fermented by the same method as in Example 1. Thereafter, the pH and sugar content of the fermented fruit juice were measured by the same method as in Example 1.

The results for the fermented apple juice are shown in Table 14.

The results for the fermented orange juice are shown in Table 15.

Since fermentation was performed three times for each strain, the "Reduction ratio," "Mannitol content" and "pH" in Tables 14 to 15 are the average values of three measurements or calculations (n=3).

**Table 14**

| Fermented Apple Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei JCM 16173^{T} | *0.73* | 0.47 | 0.31 | 0.40 | 62.05 | 26.76 | 3.87 |
| Apilactobacillus kunkeei CP3735 | 059 | 0.50 | 0.35 | 0.42 | 61.02 | 30.06 | 3.80 |
| Apilactobacillus kunkeei CP3736 | 0.42 | 0.52 | 0.37 | 0.41 | 60.03 | 30.25 | 3.70 |
| Lactiplantibacillus plantarum | 0.10 | 0.22 | 0.07 | 0.11 | 92.66 | 0.00 | 3.83 |
| Leuconostoc citreum | 0.48 | 0.18 | 0.16 | 0.21 | 82.19 | 12.77 | 4.23 |

**Table 15**

| Fermented Orange Juice | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei JCM 16173^{T} | 0.09 | 0.32 | 0.49 | 0.27 | 55.81 | 16.07 | 3.67 |
| Apilactobacillus kunkeei CP3735 | 0.07 | 0.30 | 0.54 | 0.28 | 55.52 | 16.47 | 3.63 |
| Apilactobacillus kunkeei CP3736 | 0.10 | 0.33 | 0.47 | 0.28 | 55.59 | 15.09 | 3.67 |
| Lactiplantibacillus plantarum | 0.01 | 0.13 | 0.12 | 0.08 | 70.76 | 0.00 | 3.60 |
| Leuconostoc citreum | 0.19 | 0.04 | 0.23 | 0.16 | 64.15 | 9.76 | 3.90 |

### [Example 5: Culturing of Various Lactic Acid Bacteria of the Genus Apilactobacillus in a Three-Sugar-Containing Medium]

In Example 5, using a medium containing the three sugars, the effect of reducing the three sugars by lactic acid bacteria of the genus Apilactobacillus was evaluated.

### [Test Strains]

The lactic acid bacteria strains of the genus Apilactobacillus shown in Table 16 were used.

**Table 16**

| Bacterial Species | Strain |
|---|---|
| Apilactobacillus kunkeei | CP3735 (Accession No.: NITE BP-04025) |
| Apilactobacillus kunkeei | CP3736 (Accession No.: NITE BP-04026) |
| Apilactobacillus kunkeei | CP3737 (Accession No.: NITE BP-04027) |
| Apilactobacillus kunkeei | CP3738 (Accession No.: NITE BP-04028) |
| Apilactobacillus kunkeei | JCM 16173^{T} (Type strain) |

### [Medium]

By modifying the GYP liquid medium generally used for culturing lactic acid bacteria, the SGFYP liquid medium (pH 6.8) shown in Table 17 below, which contains equal amounts of sucrose, glucose and fructose as carbon sources, was prepared.

**Table 17**

| SGFYP Liquid Medium | Content (per 100 mL of medium) |
|---|---|
| Sucrose | 0.5 g |
| Glucose | 0.5 g |
| Fructose | 0.5 g |
| Yeast extract | 1 g |
| Peptone | 0.5 g |
| Beef extract | 0.2 g |
| Sodium acetate trihydrate | 0.2 g |
| MgSO₄·7H₂O | 20 mg |
| MnSO₄·4H₂O | 1 mg |
| FeSO₄·7H₂O | 1 mg |
| NaCl | 1 mg |
| Tween 80 | 2.5 mg |
| Water | 100 ml |

### [Culturing]

To the aforementioned liquid medium, a lactic acid bacteria suspension prepared according to the same method as in Example 1 was added (inoculation amount is 5% relative to the volume of the medium), and cultured at 30°C for 18 to 22 hours. For the supernatant obtained by subjecting the culture broth to centrifugation, the pH and sugar content were measured by the same method as in Example 1. The results are shown in Table 18.

**Table 18**

| Medium | Reduction Ratio | | | | Total Content of the Three Sugars after Fermentation (mg/mL) | Mannitol (mg/mL) | pH |
|---|---|---|---|---|---|---|---|
| | Sucrose | Glucose | Fructose | The three sugars | | | |
| Apilactobacillus kunkeei CP3735 | 0.71 | 0.82 | 0.86 | 0.79 | 3.00 | 5.20 | 4.10 |
| Apilactobacillus kunkeei CP3736 | 0.92 | 0.94 | 0.89 | 0.92 | 1.22 | 5.59 | 4.03 |
| Apilactobacillus kunkeei CP3737 | 1.00 | 1.00 | 0.94 | 0.98 | 0.28 | 5.88 | 4.03 |
| Apilactobacillus kunkeei CP3738 | 0.98 | 0.89 | 0.90 | 0.93 | 1.06 | 5.94 | 4.06 |
| Apilactobacillus kunkeei JCM 16173^{T} | 0.84 | 0.89 | 0.91 | 0.88 | 1.76 | 6.58 | 4.14 |

All five strains of Apilactobacillus kunkeei consumed the three sugars of sucrose, glucose and fructose in the medium and reduced their contents (Table 16). From this result, it is considered that the effect of simultaneously reducing the contents of the three sugars by lactic acid bacteria of the genus Apilactobacillus is not limited to specific fruit juices such as apple juice or orange juice, but can also be obtained for plant-derived compositions containing the three sugars.

### [Example 6: Sensory Evaluation of Lactic Acid Bacteria Fermented Fruit Juice]

### [Test Strains]

The lactic acid bacteria strains shown in Table 19 were used.

**Table 19**

| Bacterial Species | Strain |
|---|---|
| Apilactobacillus kunkeei | CP3736 (Accession No.: NITE BP-04026) |
| Lactiplantibacillus plantarum | NRIC 1067^{T} (Type strain) |
| Fructobacillus fructosus | NRIC 1058^{T} (Type strain) |

### [Plant-Derived Composition (Fermentation Raw Material)]

1N NaOH was added to straight apple juice (from Nagano Prefecture) to adjust the pH to 5.5. To this, yeast extract (product name: YEASTOCK S-Pd (Asahi Group Foods, Ltd.)) was added as a fermentation aid (final concentration 0.1%) and used as a fermentation raw material.

The contents of the three sugars (glucose, fructose, and sucrose) and mannitol, pH and Brix value in the fermentation raw material are shown in Table 20.

**Table 20**

| Plant-Derived Composition (Fermentation Raw Material) | Content (g/100mL) | | | | Mannitol (mg/mL) | Brix Value | pH |
|---|---|---|---|---|---|---|---|
| | Sucrose | Glucose | Fructose | The three sugars | | | |
| Apple juice | 2.22 | 3.10 | 8.19 | 13.51 | 0.00 | 10 | 5.50 |

### [Lactic Acid Bacteria Fermentation]

To 100 mL of the aforementioned fermentation raw material, 5 mL of a lactic acid bacteria suspension prepared according to the method described in Example 1 was added (inoculation amount is 5% relative to the volume of the fermentation raw material), and fermented at 30°C for 20 hours (static culture). Thereafter, for the fermented fruit juice obtained using Apilactobacillus kunkeei (strain CP3736), the pH and sugar content were measured by the same method as in Example 1. The results are shown in Table 21. For this strain, fermentation was performed three times, so the "Reduction ratio," "Mannitol content" and "pH" in Table 21 are the average values of three measurements or calculations (n=3).

**Table 21**

| Fermented Apple Juice | Reduction Ratio | | | | Mannitol (mg/mL) | Brix Value | pH |
|---|---|---|---|---|---|---|---|
| | Sucrose | Glucose | Fructose | The three sugars | | | |
| Apilactobacillus kunkeei (strain CP3736) | 0.42 | 0.40 | 0.31 | 0.35 | 14.61 | - | 3.94 |

The fermented fruit juice was subjected to centrifugation (9000 x g, 10 minutes), and 90 mL of the recovered supernatant was sterilized by heating at 65°C for 10 minutes. A sensory evaluation was conducted on the sterilized fermented fruit juice.

The evaluation was conducted blindly by four trained panelists with three or more years of experience in sensory evaluation of beverages. Each panelist drank 20 mL of each evaluation sample in a 90 mL commercially available plastic cup to perform the evaluation.

The evaluation items were "mellowness," "refreshing feeling," "richness," "aroma," "sourness," and "overall flavor."

For each evaluation item, the evaluation score for the straight apple juice (without pH adjustment and addition of fermentation aid) used to prepare the fermentation raw material was set to 5. If each evaluation item was improved by fermentation, the evaluation score was set to 6 to 10 (the highest score is 10), and if it was worsened by fermentation, the evaluation score was set to 1 to 4 (the lowest score is 1). The results are shown in Table 22. The evaluation scores in Table 22 are the average values of the evaluation scores by the four panelists (n=4).

**Table 22**

| Evaluation Item | Apilactobacillus kunkeei | Lactiplantibacillus plantarum | Fructobacillus fructosus |
|---|---|---|---|
| Mellowness | 2.3 | 3.5 | 4.3 |
| Refreshing feeling | 8 | 7.8 | 5 |
| Richness | 2.8 | 2.8 | 4 |
| Aroma | 3.5 | 4.3 | 2.3 |
| Sourness | 8.3 | 6.5 | 5.3 |
| Overall flavor | 4.8 | 4.3 | 2 |

The apple juice fermented with lactic acid bacteria of the genus Apilactobacillus had increased sourness and refreshing feeling compared to before fermentation. Also, its overall flavor was the highest among the three bacterial species evaluated.

### Industrial Applicability

The present invention is applicable to food and drink, etc.

## Claims

1. A fermented product of a plant-derived composition with lactic acid bacteria of the genus Apilactobacillus, containing mannitol.

2. The fermented product according to claim 1, wherein the content of mannitol is 1.5 mg/mL or more.

3. The fermented product according to claim 1, wherein
a total content (A) of glucose, fructose, and sucrose in the plant-derived composition before fermentation and
a total content (B) of glucose, fructose, and sucrose in the fermented product
have the following relationship:$\left(A-B\right) / A = 0.15 to 1.00$

4. The fermented product according to claim 1, wherein a total content of glucose, fructose, and sucrose is 500 mg/mL or less.

5. The fermented product according to claim 1, wherein the lactic acid bacteria of the genus Apilactobacillus are selected from the group consisting of:
Apilactobacillus kunkeei,
Apilactobacillus apinorum,
Apilactobacillus micheneri, and
Apilactobacillus timberlakei.

6. The fermented product according to claim 1, wherein the lactic acid bacteria of the genus Apilactobacillus are selected from the group consisting of:
Apilactobacillus quenuiae,
Apilactobacillus ozensis,
Apilactobacillus nanyangensis,
Apilactobacillus zhangqiuensis, and
Apilactobacillus xinyiensis.

7. The fermented product according to claim 1, wherein the lactic acid bacteria of the genus Apilactobacillus are Apilactobacillus kunkeei.

8. The fermented product according to claim 1, wherein the lactic acid bacteria of the genus Apilactobacillus are selected from the group consisting of:
Apilactobacillus kunkeei strain CP3735 (Accession No. NITE BP-04025),
Apilactobacillus kunkeei strain CP3736 (Accession No. NITE BP-04026),
Apilactobacillus kunkeei strain CP3737 (Accession No. NITE BP-04027), and
Apilactobacillus kunkeei strain CP3738 (Accession No. NITE BP-04028).

9. The fermented product according to claim 1, wherein the pH is 5 or less.

10. The fermented product according to claim 1, wherein the plant-derived composition comprises vegetable juice or fruit juice.

11. The fermented product according to claim 10, wherein the plant-derived composition comprises apple or orange fruit juice.

12. A food or drink composition, comprising the fermented product according to claim 1.

13. A fermented product, which is obtained by fermenting a plant-derived composition with lactic acid bacteria, wherein
the plant-derived composition before fermentation contains glucose, fructose, and sucrose,
the lactic acid bacteria are lactic acid bacteria of the genus Apilactobacillus, and
the fermented product contains mannitol.

14. A method for producing a lactic acid bacteria fermented product of a plant-derived composition, comprising
a step of fermenting a plant-derived composition containing glucose, fructose, and sucrose with lactic acid bacteria of the genus Apilactobacillus,
wherein the fermented product contains mannitol.

15. A method for reducing a sugar content of a plant-derived composition, comprising
a step of fermenting a plant-derived composition containing a sugar with lactic acid bacteria of the genus Apilactobacillus,
wherein the sugar is glucose, fructose, and sucrose.
